## Europäisches Patentamt

## European Patent Office

## Office européen des brevets

(19)

(11) Veröffentlichungsnummer: **0 037 891**
**B1**

(12) **EUROPÄISCHE PATENTSCHRIFT**

(45) Veröffentlichungstag der Patentschrift:
**14.12.83**

(51) Int. Cl.³: **C 07 C 149/18, C 07 C 148/00, B 01 J 21/02, B 01 J 29/06, B 01 J 23/02**

(21) Anmeldenummer: **81101252.5**

(22) Anmeldetag: **21.02.81**

(54) Verfahren zur Herstellung von 3-Mercapto-propandiol-(1,2).

(30) Priorität: **12.04.80 DE 3014165**

(43) Veröffentlichungstag der Anmeldung:
**21.10.81 Patentblatt 81/42**

(45) Bekanntmachung des Hinweises auf die Patenterteilung:
**14.12.83 Patentblatt 83/50**

(84) Benannte Vertragsstaaten:
**BE CH DE FR GB IT LI NL**

(56) Entgegenhaltungen:
**CH - A - 574 406**
**DE - A - 1 801 551**
**DE - A - 2 129 162**
**DE - A - 2 209 458**

(73) Patentinhaber: **Degussa Aktiengesellschaft,
Weissfrauenstrasse 9, D-6000 Frankfurt am Main 1 (DE)**

(72) Erfinder: **Kleemann, Axel, Dr., Greifenhagenstrasse 25,
D-6450 Hanau 9 (DE)**
Erfinder: **Nygren, Robert, Dr., Fürstenbergstrasse 8,
D-6450 Hanau 9 (DE)**
Erfinder: **Wagner, Rudolf, Dr., Greifenhagenstrasse 9,
D-6450 Hanau 9 (DE)**

Verfahren zur Herstellung von 3-Mercapto-propandiol-(1,2)

In der Literatur sind bis heute zwei Verfahren beschrieben worden, nach denen 3-Mercapto-propandiol-(1,2) — auch Monothioglycerin genannt — durch Addition von Schwefelwasserstoff an Glycid hergestellt werden kann.

Nach L. Smith und B. Sjöberg (Ber. deutsch. chem. Ges. *69*, 678 (1936) und Ber. deutsch. chem. Ges. *75*, 13, (1942) wird 1/3 Mol Glycid zu einer mit Schwefelwasserstoff gesättigten Lösung von 1/3 Mol Bariumhydrogensulfid unter laufendem Einleiten von Schwefelwasserstoff zugetropft. Die Ausbeute nach diesem Verfahren beträgt im Höchstfall 61 % d. Th., wobei angegeben wird, dass bei Vergrösserung des Ansatzes über 1/3 Mol hinaus die Ausbeute abfällt. Daneben entstehen höhermolekulare Kondensationsprodukte des Glycids. Durch Destillation kann Monothioglycerin von Wasser und den Nebenprodukten getrennt werden. Aufgrund der Schwierigkeiten bei der Vergrösserung der Ansätze stellt diese Herstellmethode für Monothioglycerin kein technisch brauchbares Verfahren dar.

In der DE-PS 910 296 wird zwar ein kontinuierliches Verfahren zur Herstellung von Monothioglycerin beschrieben, wobei Ausbeuten von etwa 80 - 95 % erzielt werden sollen.

Hierbei bedient man sich einer Umlaufapparatur, welche mit wässrigem Alkohol, z.B. Isopropanol, und katalytischen Mengen, z.B. 0,3 %, Alkali- oder Erdalkalihydrogensulfid, wie Calciumhydrogensulfid, beschickt wird. In die Apparatur wird gasförmiger Schwefelwasserstoff so eingeleitet, dass ständig in der gesamten Apparatur bzw. Lösung Schwefelwasserstoff in Form feinverteilter Gasblasen vorhanden und seine Konzentration an der Einleitungsstelle des Glycids am grössten ist. Die Temperatur des Reaktionsgemisches wird zwischen 20 und 35°C gehalten. Aus der Umlaufapparatur wird das Reaktionsgemisch kontinuierlich abgezogen.

Durch Einleiten von Kohlendioxid wird der Katalysator als Carbonat gefällt, die Lösung anschliessend filtriert und der eingesetzte Alkohol destillativ zurückgewonnen.

In einer nächsten Destillationsstufe wird das Wasser entfernt und der Rückstand schliesslich einer Fraktionierung im Vakuum unterworfen, wobei man dann das Monothioglycerin erhält. In allen Beispielen wird der Katalysator vor der Destillation des Produktes aus dem Rohprodukt entfernt, wodurch die Destillation des Produktes ohne Schwierigkeiten verlaufen soll.

Das bekannte Verfahren ist einmal an eine spezielle Apparatur gebunden, da ständig genügend Schwefelwasserstoff zum Abfangen des aus dem Hydrogensulfid gebildeten Hydroxids vorhanden sein muss. Anderenfalls treten Kondensationsreaktionen zwischen Glycid und dem gebildeten Monothioglycerin auf.

Ausserdem muss der eingesetzte Katalysator nach Beendigung der Reaktion durch Einleiten von Kohlendioxid in ein Carbonat überführt werden, das als solches nicht wieder einsatzfähig ist.

Ferner darf die Destillation des Rohproduktes nicht in Gegenwart des Katalysatorcarbonates durchgeführt werden.

Zweck des erfindungsgemässen Verfahrens ist daher ein technisch einfach durchzuführendes Verfahren, das ebenfalls zu hohen Ausbeuten führt.

Es wurde nun gefunden, dass sich Monothioglycerin in hohen Ausbeuten in technisch einfacher Weise herstellen lässt, wenn man flüssigen Schwefelwasserstoff mit Glycid in Anwesenheit eines Katalysators in heterogener oder homogener Phase reagieren lässt.

Als feste Katalysatoren, die sich nicht im Reaktionsmedium lösen, eignen sich besonders gut schwach basische Katalysatoren wie Aluminiumoxide, z.B. ein aktiviertes Aluminiumoxid mit einem Alkaligehalt von 0,02 bis 5 Gew.-%, Natriumaluminiumsilikate wie Zeolithe und Hydroxysodalithe. Diese festen Katalysatoren zeichnen sich durch eine hohe Standzeit aus. Sie werden bevorzugt als Festbettkatalysatoren eingesetzt. Ein sehr geeigneter Zeolith ist z.B. Montmorillonit. Als Katalysatoren zum Arbeiten in homogener Phase eignen sich Alkalioder Erdalkalihydroxide, die sich in dem Reaktionsmedium aus Glycid und flüssigem Schwefelwasserstoff lösen. Bevorzugt sind Natrium- und Kaliumhydroxid.

Das Verfahren wird unter Drücken von 15 bis 200 bar durchgeführt.

Die Temperaturen liegen in der heterogenen Phase am Festbett zwischen 30 und 150°C, in der homogenen Phase im allgemeinen an der oberen genannten Temperaturgrenze bzw. noch etwas höher. Im allgemeinen kommen Temperaturen von 30 bis 180°C in Frage.

Das Molverhältnis von flüssigem Schwefelwasserstoff zu Glycid liegt bei 3 : 1 bis 10 : 1, bevorzugt bei 4 : 1 bis 6 : 1, unabhängig, ob in heterogener oder homogener Phase gearbeitet wird.

Das Verfahren kann sowohl kontinuierlich wie diskontinuierlich durchgeführt werden.

Beispielhaft wird es näher an einer kontinuierlichen Durchführung mit Festbettkatalysator erläutert:

Über zwei Pumpen dosiert man flüssigen Schwefelwasserstoff und Glycid in dem gewählten Molverhältnis über einen mit dem Katalysator gefüllten Festbettreaktor.

Der Druck, der nötig ist, um Schwefelwasserstoff in flüssiger Phase zu halten, wird durch ein Druckhalteventil am Ausgang des Festbettreaktors eingestellt.

Die gewünschte Temperatur im Festbettreaktor wird durch Wärmeabfuhr über den Aussenmantel gehalten.

Das Reaktionsgemisch wird über das Druckhalteventil auf Atmosphärendruck entspannt. Hierbei entweicht der überschüssige Schwefelwasserstoff nahezu quantitativ; er kann nach einer anschliessenden Kondensation dem Reaktionssystem wieder zugeführt werden.

Das zurückbleibende Reaktionsprodukt, das frei von Katalysator ist und nur noch etwa 0,25 Gew.-%

Schwefelwasserstoff enthält, wird anschliessend unter Vakuum destillativ gereinigt.

Es zeigt sich, dass bei einem Molverhältnis von «Schwefelwasserstoff : Glycid» unter 3 : 1 die Ausbeute an Monothioglycerin stark absinkt und sich im selben Masse die Bildung des Bisadduktes von Glycid an Schwefelwasserstoff Bis-(2,3-dihydroxypropyl)-sulfid steigert. Dem Molverhältnis «Schwefelwasserstoff : Glycid» ist an und für sich nach oben keine Grenze gesetzt. Es zeigte sich jedoch, dass ab einem Molverhältnis von 6 : 1 keine wesentliche Ausbeutesteigerung mehr eintritt, wohl aber ein Absinken der Raum-Zeitausbeute.

Der Temperaturbereich, bei dem die Reaktion durchgeführt wird, sollte unter 150°C gewählt werden — wenn in heterogener Phase gearbeitet wird — da oberhalb dieser Temperatur in heterogener Phase merkliche Eigenkondensation von Glycid zu immer stärkeren Ausbeuteverlusten führt. Die Reaktionstemperatur sollte jedoch nicht unter 30°C gewählt werden, damit die Addition mit wirtschaftlich sinnvoller Geschwindigkeit abläuft. Zwischen 30 und 150°C konnte kein Temperatureinfluss auf die Ausbeute an Monothioglycerin in heterogener Phase festgestellt werden.

In homogener Phase dagegen ist es auch möglich, 150°C zu überschreiten und noch eine Ausbeute von etwa 81% der Theorie zu erhalten, siehe Beispiel 10.

Wie gesagt, ist man bei der Durchführung der Reaktion nicht auf die oben beschriebene kontinuierliche Arbeitsweise beschränkt. Die Addition lässt sich ebenso diskontinuierlich in Autoklaven oder sonstigen Druckreaktoren durchführen.

Bei homogener Katalyse lassen sich ebenfalls Ausbeuten bis zu 95% der Theorie erreichen, siehe Beispiel 8. Bei einer Temperaturerhöhung über 150°C sinkt jedoch die Ausbeute, erreicht aber — wie schon gesagt — immer noch Werte um 81%.

Bei homogener Katalyse erhält man ein Rohprodukt, das den Katalysator in gelöster Form enthält. Überraschenderweise störte das Vorhandensein des Katalysators im Rohprodukt bei dessen destillativer Aufarbeitung nicht und führte somit zu keiner Ausbeuteeinbusse. Die Anwendung der homogenen Katalyse eignet sich besonders für diskontinuierliche Arbeitsweise in Autoklaven oder Rührbehältern.

Schliesslich ist es auch möglich, die Umsetzung zwischen flüssigem Schwefelwasserstoff und Glycid in Gegenwart geringer Mengen Lösungsmittel wie Wasser oder niederen aliphatischen Alkoholen wie Methanol, Äthanol, den Propanolen durchzuführen. Glycid und Lösungsmittel werden im Gewichtsverhältnis 1 : 0,5 bis 1 : 5 verwendet.

Monothioglycerin ist ein technisch interessanter Synthesebaustein und kann eingesetzt werden
— in der Haarkosmetik, siehe US-PS 3 415 606
— in Enthaarungsmitteln, s. DE-OS 2 253 117
— als Schutzmittel gegen UV- und Röntgenstrahlen, siehe Protoplasma 45, 293
— zur Stabilisierung von Arzneimittelzubereitungen, siehe US-PS 3 026 248
— in photographischen Entwicklerlösungen, siehe FR-PS 1 410 426
— als Farbstabilisator in Polymeren, siehe US-PS 2 560 053
— als Enzymaktivator in enzymhaltigen Waschmitteln, siehe DE-OS 1 953 816.

*Beispiel 1:*

Ein 2 Ltr.-Edelstahlautoklav wird mit 10 g Aluminiumoxid (Kugelform, $\varnothing$ 2-4 mm, BET = 300 m$^2$/g, Na$_2$O = 0,08 Gew.-%), 1020 g flüssigem Schwefelwasserstoff (30 Mol) und 444 g Glycid (6 Mol) beschickt. Das Gemisch wird unter Rühren 4 Stunden auf 70°C erhitzt.

Anschliessend wird der Autoklav entspannt und der flüssige Inhalt einer Vakuumdestillation unterworfen. Bei 89° und 0,9 mbar erhält man 614,5 g 3-Mercapto-propandiol-(1,2), entsprechend 94,8% der Theorie, Siedepunkt $\geqq$ 95°C (1 Torr), Reinheit $\geqq$ 99,5 Gew.-% (jodometrische Titration).

*Beispiel 2:*

Ein 2 Ltr.-Edelstahlautoklav wird mit 50 g pulverförmigem Zeolith (Montmorillonit), 1020 g flüssigem Schwefelwasserstoff (30 Mol) und 444 g Glycid (6 Mol) beschickt. Das Gemisch wird unter Rühren 4 Stunden auf 30°C erhitzt.

Anschliessend wird der Autoklav entspannt und der flüssige Inhalt einer Vakuumdestillation unterworfen. Man erhält 591 g 3-Mercapto-propandiol-(1,2), entsprechend 91,2% der Theorie. Siedepunkt und Reinheit wie in Beispiel 1.

*Beispiel 3:*

Ein 2 Ltr.-Edelstahlautoklav wird mit 50 g pulverförmigem Hydroxysodalith, 1020 g flüssigem Schwefelwasserstoff (30 Mol) und 444 g Glycid (6 Mol) beschickt. Das Gemisch wird unter Rühren 4 Stunden auf 70°C erhitzt. Nach destillativer Aufarbeitung erhält man 587 g 3-Mercapto-propandiol-(1,2), entsprechend 90,6% der Theorie. Siedepunkt und Reinheit wie in Beispiel 1.

*Beispiel 4:*

Ein 2 Ltr.-Edelstahlautoklav wird mit 5 g Aluminiumoxid (Kugelform, $\varnothing$ 4-6 mm, BET = 250 m$^2$/g, Na$_2$O = 3 Gew.-%), 1020 g flüssigem Schwefelwasserstoff und 444 g Glycid (6 Mol) beschickt. Das Gemisch wird unter Rühren 4 Stunden auf 70°C erhitzt. Nach destillativer Aufarbeitung des erhaltenen Rohproduktes erhält man 616 g 3-Mercapto-propandiol-(1,2), entsprechend 95,1% der Theorie.

*Beispiel 5:*

Der in Beispiel 4 genannte Versuch wird unter genau den gleichen Bedingungen 10mal wiederholt, jedoch wird der gleiche Katalysator ohne Ersatz oder Regeneration, also in gebrauchtem Zustand, immer wieder von neuem eingesetzt. Folgende Ergebnisse wurden erzielt:

| Versuch | | Ausbeute [g] | Ausbeute [% d.Th.] |
|---|---|---|---|
| Wiederholung | 1 | 615 | 94,9 |
| » | 2 | 618 | 95,3 |
| » | 3 | 616 | 95,0 |
| » | 4 | 605 | 93,3 |
| » | 5 | 617 | 95,2 |
| » | 6 | 612 | 94,5 |
| » | 7 | 614 | 94,7 |
| » | 8 | 608 | 93,8 |
| » | 9 | 614 | 94,7 |
| » | 10 | 613 | 94,6 |

Nach elfmaligem Einsatz zeigt also der Katalysator — nämlich Aluminiumoxid entsprechend Beispiel 1 — keine Einbusse in seiner Aktivität.

*Beispiel 6:*

Ein 2 Ltr.-Edelstahlautoklav wird mit 5 g Aluminiumoxid — entsprechend Beispiel 1 — 1020 g flüssigem Schwefelwasserstoff (30 Mol) und 222 g Glycid (3 Mol) beschickt. Das Gemisch wird unter Rühren 4 Stunden auf 30°C erhitzt. Nach destillativer Aufarbeitung erhält man 309 g 3-Mercapto-propandiol-(1,2), entsprechend 95,3% der Theorie.

*Beispiel 7:*

Ein 2 Ltr.-Edelstahlautoklav wird mit 1 g Kaliumhydroxid, 1020 g flüssigem Schwefelwasserstoff (30 Mol) und 444 g Glycid (6 Mol) beschickt. Das Gemisch wird unter Rühren 4 Stunden auf 70°C erhitzt. Nach destillativer Aufarbeitung erhält man 612 g 3-Mercapto-propandiol-(1,2), entsprechend 94,5% der Theorie.

*Beipsiel 8:*

Ein 2 Ltr.-Edelstahlautoklav wird mit 1 g Natriumhydroxid, 1020 g flüssigem Schwefelwasserstoff (30 Mol) und 444 g Glycid (6 Mol) beschickt. Das Gemisch wird 30 Minuten auf 145°C erhitzt. Nach destillativer Aufarbeitung erhält man 605 g 3-Mercapto-propandiol-(1,2), entsprechend 93,3% der Theorie.

*Beispiel 9:*

Ein 2 Ltr.-Edelstahlautoklav wird mit 1 g Kaliumhydroxid, 1020 g flüssigem Schwefelwasserstoff (30 Mol) und 444 g Glycid (6 Mol) beschickt. Das Gemisch wird 30 Minuten auf 170°C erhitzt. Nach destillativer Aufarbeitung erhält man 521 g 3-Mercapto-propandiol-(1,2), entsprechend 80,5% der Theorie.

*Beispiel 10:*

In ein Doppelmantelrohr, dessen inneres Volumen 4,2 Ltr. beträgt und mit Aluminiumoxid, entsprechend Beispiel 1, gefüllt ist, werden über zwei Pumpen pro Stunde 1020 g flüssiger Schwefelwasserstoff und 444 g Glycid (6 Mol) dosiert. Die Temperatur im Inneren des Reaktors wird mit Hilfe von Warmwasser, welches durch den äusseren Mantel gepumpt wird, auf 50°C gehalten. Der zur Flüssighaltung des Reaktionsgemisches notwendige Druck (35 bar) wird durch ein Druckhalte-Regelventil gehalten, an welchem das Reaktionsgemisch nach Durchlaufen des Reaktors entspannt wird. Das nach zehnstündigem Lauf der Apparatur erhaltene Rohprodukt ergab nach destillativer Aufarbeitung 6,17 kg Monothioglycerin, entsprechend einer Ausbeute von 95,2% der Theorie.

Das Reaktorvolumen wurde also so gewählt, dass die Komponente im Unterschuss, nämlich Glycid, zu 100% wegreagierte.

Auch in den Beispielen 4-10 entsprachen der Siedepunkt und die Reinheit des Produktes den Angaben in Beispiel 1.

**Patentansprüche**

1. Verfahren zur Herstellung von 3-Mercapto-propandiol-(1,2) durch Umsetzung von Schwefelwasserstoff mit Glycid in Gegenwart von Katalysatoren, dadurch gekennzeichnet, dass man flüssigen Schwefelwasserstoff mit Glycid im Molverhältnis von 3 : 1 bis 10 : 1, bevorzugt von 4 : 1 bis 6 : 1, unter einem Druck von 15 bis 200 bar miteinander umsetzt, so dass der Schwefelwasserstoff in flüssigem Zustand verbleibt, wobei entweder in heterogener Phase mit dem Katalysator in fester Form bei 30 - 150°C oder in homogener Phase mit dem im Reaktionsmedium gelösten Katalysator bei 30 - 180°C gearbeitet wird.

2. Verfahren nach Anspruch 1, dadurch gekennzeichnet, dass man bei Durchführung des Verfahrens in heterogener Phase ein Aluminiumoxid oder ein Natriumaluminiumsilikat einsetzt.

3. Verfahren nach Anspruch 1 und 2, dadurch gekennzeichnet, dass man als Natriumaluminiumsilikat einen Zeolith oder Hydroxysodalith einsetzt.

4. Verfahren nach Anspruch 1, dadurch gekennzeichnet, dass man bei Durchführung der Umsetzung in homogener Phase Alkalihydroxide oder Erdalkalihydroxide in das Reaktionsmedium einführt.

5. Verfahren nach Anspruch 1-4, dadurch gekennzeichnet, dass man die Umsetzung in Anwesenheit einer geringen Menge Lösungsmittel wie Wasser oder niedermolekularem aliphatischen Alkohol durchführt.

**Claims**

1. A process for the production of 3-mercapto-propan-1,2-diol by the reaction of hydrogen sulphide with glycidol in the presence of catalysts, characterised in that liquid hydrogen sulphide is reacted with glycidol in a molar ratio of from 3 : 1 to 10 : 1, preferably from 4 : 1 to 6 : 1, under a pressure of from 15 to 200 bars so that the hydrogen sulphide remains in a liquid condition, the process being carried out either in heterogeneous phase with the catalyst in solid form at from 30 to 150°C, or in homogeneous phase with the catalyst dissolved in the reaction medium, at from 30 to 180°C.

2. A process according to claim 1, characterised in that when carrying out the process in heterogeneous phase, an aluminium oxide or a sodium aluminium silicate is used.

3. A process according to claims 1 and 2, characterised in that a zeolite or hydroxy sodalite is used as sodium aluminium silicate.

4. A process according to claim 1, characterised in that when carrying out the reaction in homogeneous phase, alkali metal hydroxides or alkaline-earth metal hydroxides are introduced into the reaction medium.

5. A process according to claims 1 to 4, characterised in that the reaction is carried out in the presence of a small quantity of solvent, such as water or low molecular weight aliphatic alcohol.

**Revendications**

1. Procédé de préparation de 3-mercapto-propanediol-(1,2) par réaction de sulfure d'hydrogène avec du glycide, en présence de catalyseurs, caractérisé en ce qu'on fait réagir le sulfure d'hydrogène liquide avec le glycide dans le rapport molaire de 3 : 1 à 10 : 1, de préférence de 4 : 1 à 6 : 1, sous une pression de 15 à 200 bars de telle sorte que le sulfure d'hydrogène reste à l'état liquide, en travaillant soit en phase hétérogène avec un catalyseur à l'état solide à une température comprise entre 30 et 150°C, soit en phase homogène avec le catalyseur dissous dans le milieu réactionnel, à une température comprise entre 30 et 180°C.

2. Procédé suivant la revendication 1, caractérisé en ce qu'on utilise pour la mise en oeuvre du procédé en phase hétérogène, un oxyde d'aluminium ou un silicate d'aluminium et de sodium.

3. Procédé suivant les revendications 1 et 2, caractérisé en ce qu'on utilise un zéolite ou un hydroxysodalite à titre de silicate d'aluminium et de sodium.

4. Procédé suivant la revendication 1, caractérisé en ce qu'on introduit dans le milieu réactionnel des hydroxydes ou alcalino-terreux, pour la mise en oeuvre de la réaction en phase homogène.

5. Procédé suivant les revendications 1 à 4, caractérisé en ce qu'on effectue la réaction en présence d'une faible quantité de solvant comme de l'eau ou un alcool aliphatique de poids moléculaire peu élevé.